# EUROPEAN PATENT APPLICATION

(11) **EP 3 449 760 A1**
(43) Date of publication of application: **06.03.2019**
(21) Application number: 18180762.9
(22) Date of filing: 29.06.2018
(51) Int. Cl.: A45C 13/28, A45C 15/06, B65D 71/52, B65D 25/28, B65D 85/00, F21V 33/00

(54) **DIAPER CADDY**

(30) Priority: 05.09.2017 US 201715695576
(71) Applicant: Skip-Hop, Inc., New York, NY 10010-5270 (US)
(72) Inventor: CARTER, Chelsea, Jersey City, NJ New Jersey 07306 (US); VILLANO, Janet, Jersey City, NJ New Jersey 07302 (US); DIAMANT, Ellen, New York, 10011 (US)
(74) Representative: Appleyard Lees IP LLP

(57) **Abstract**

A diaper caddy is provided including a basket having an open top and a handle spanning the open top of the basket with a light source on the handle that directs light into the open top of the basket and around the basket.

## Description

### FIELD OF INVENTION

The present invention relates to a diaper caddy, and in particular, in one form, the diaper caddy is an open basket with a handle that includes a light.

### BACKGROUND OF THE INVENTION

Diaper caddies are products used to store, organize, and/or carry various items needed to change an infant's diaper. Typical diaper caddies include various storage compartments to help a user find a particular desired item and oftentimes include a handle that assist in carrying the diaper caddy.

What is needed in the art is a diaper caddy with features that improve the overall process of changing an infant's diaper.

### SUMMARY OF THE INVENTION

The present invention relates to a diaper caddy, and in particular, in one form, the diaper caddy is an open basket with a handle that includes a light. One exemplary diaper caddy made in accordance with the present invention includes a basket with sides that define an open top of the basket. A handle is connected to the right side and the left side of the basket so as to span across the open top.

The handle includes a light that illuminates the interior of the basket by directing light into the open top of the basket. In particular, the light comprises a light source, such as an LED bulb, which is surrounded by a diffuser which projects below the bottom surface of the handle. Accordingly, the light not only directs light into the basket, but with the diffuser, the light also directs light around the basket. The light is therefore able to provide additional visibility to the surroundings. Advantageously, a user can not only see the contents within the basket, but also the surroundings to assist, for example, in changing a diaper. Furthermore, in one particular embodiment, the light only produces about 16 lumens, and because of this relatively low level of lumens, the light is less likely to disturb a tired or sleeping infant.

The light is turned on by use of a switch positioned on the top of the handle, which in some embodiments, is a touch switch. In this way, there is no clicking noise created when the switch is activated and the light is turned on/off, thus reducing the potential of disturbing a tired or sleeping infant. It is further contemplated that the light also automatically turns off after a predetermined time. For example, in some embodiments, the light turns off after about 10 minutes.

The exemplary diaper caddy of the present invention is designed to hold at least 30 lbs, but is preferably made of lightweight, semi-rigid material such that the diaper caddy makes minimal noise when set down or moved. To this end, the sides of the basket are preferably made of a thin polyethylene board surrounded by a fabric liner. The polyethylene board is able to flex and partially deform while still providing sufficient strength to substantially maintain the overall shape of the basket when loaded, and the fabric liner provides sound damping as well as an aesthetically appealing finish.

As previously mentioned, an insert is positioned within the basket, and, in this exemplary embodiment, the insert is operably connected to the right side and the left side along the interior surface of the basket. In particular, a hook-and-loop fastener is used to removably secure the insert to the interior surface of the sides of the basket.

One of ordinary skill in the art will recognize that additional embodiments are also possible without departing from the teachings of the presently-disclosed subject matter. This detailed description, and particularly the specific details of the exemplary embodiments disclosed herein, is given primarily for clarity of understanding, and no unnecessary limitations are to be understood therefrom, for modifications will become apparent to those skilled in the art upon reading this disclosure and can be made without departing from the spirit and scope of the presently-disclosed subject matter.

Aspects (and various related combinations of features) of invention are described below with reference to the following paragraphs:
Paragraph 1. A diaper caddy, comprising:
   a basket having an open top; and
   a handle spanning the open top of the basket, the handle including a light source that directs light into the open top of the basket and around the basket.
Paragraph 2. The diaper caddy of paragraph 1, wherein the handle further includes a diffuser positioned adjacent to the light source, the diffuser configured to direct a portion of the light around the basket.
Paragraph 3. The diaper caddy of paragraph 2, wherein the diffuser projects below a bottom surface of the handle.
Paragraph 4. The diaper caddy of paragraph 1, wherein the light source produces about 16 lumens.
Paragraph 5. The diaper caddy of paragraph 1, wherein the handle further includes a touch switch that turns on the light source.
Paragraph 6. The diaper caddy of paragraph 1, wherein the light source automatically shuts off after a predetermined time.
Paragraph 7. The diaper caddy of paragraph 6, wherein the predetermined time is about 10 minutes.
Paragraph 8. The diaper caddy of paragraph 1, wherein the basket is comprised of a semi-rigid material surrounded by a fabric liner.
Paragraph 9. The diaper caddy of paragraph 8, wherein the handle is comprised of a rigid material.
Paragraph 10. The diaper caddy of paragraph 1, further comprising an insert positioned within the basket, the insert removably secured to the basket.
Paragraph 11. The diaper caddy of paragraph 10, wherein the insert is removably secured to two sides of the basket by hook-and-loop fasteners.
Paragraph 12. A diaper caddy, comprising:
   a basket having an open top; and
   a handle spanning the open top of the basket, the handle including a light source that directs light into the open top of the basket and a diffuser positioned adjacent to the light source to direct a portion of the light around the basket.
Paragraph 13. The diaper caddy of paragraph 12, wherein the light source produces about 16 lumens.
Paragraph 14. The diaper caddy of paragraph 12, wherein the handle further includes a touch switch that turns on the light source.
Paragraph 15. The diaper caddy of paragraph 12, wherein the basket is comprised of a semi-rigid material surrounded by a fabric liner.
Paragraph 16. The diaper caddy of paragraph 15, wherein the handle is comprised of a rigid material.
Paragraph 17. The diaper caddy of paragraph 12, further comprising an insert positioned within the basket, the insert removably secured to the basket.
Paragraph 18. A diaper caddy, comprising:
   a basket having an open top; and
   a handle spanning the open top of the basket, the handle including a light source that directs light into the open top of the basket, a diffuser positioned adjacent to the light source to direct a portion of the light around the basket, and a touch switch that turns on the light source; and
   wherein the light source produces about 16 lumens.
Paragraph 19. The diaper caddy of paragraph 18, wherein the basket is comprised of a semi-rigid material surrounded by a fabric liner.
Paragraph 20. The diaper caddy of paragraph 18, further comprising an insert positioned within the basket, the insert removably secured to the basket.

### BRIEF DESCRIPTION OF THE DRAWINGS

The present invention will now be described with regard to the drawings as follows.
FIG. 1 is a front, left perspective view of a diaper caddy made in accordance with the present invention.
FIG. 2 is a front, right perspective view of the removable insert of the diaper caddy of FIG. 1 shown in isolation.
FIG. 3 is a top view of the diaper caddy of FIG. 1.
FIG. 4 is a front view of the diaper caddy of FIG. 1.

### DETAILED DESCRIPTION

The present invention will now be described with reference to the Figures. It will be appreciated that other diaper caddy designs and appearances are possible which are consistent with the described function of the diaper caddy.

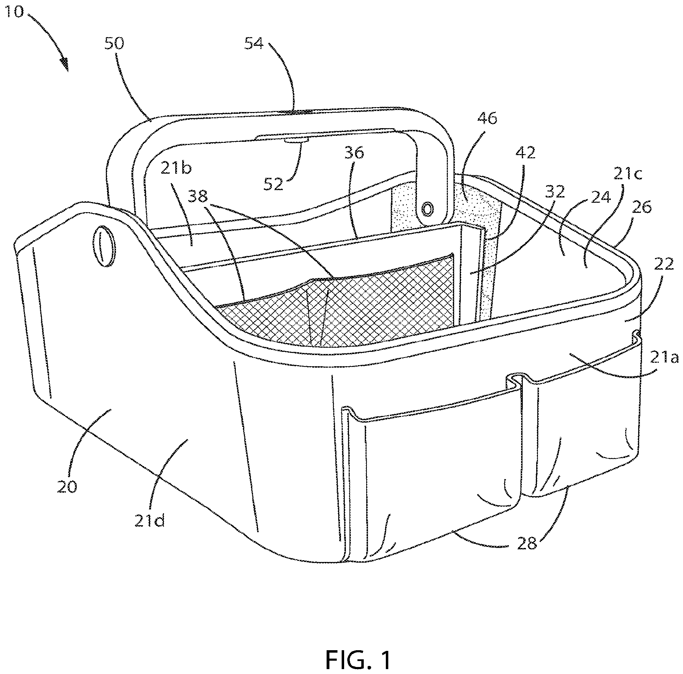

Referring first to FIG. 1, a diaper caddy 10 made in accordance with the present invention includes a basket 20 with sides 21 (i.e., a front side 21a, a back side 21b, a right side 21c, and a left side 21d) that define an open top 26 of the basket 20. A handle 50 is connected to the right side 21c and the left side 21d of the basket 20 so as to span across the open top 26, and a removable insert 30 forms an adjustable (movable) partition within the basket 20, as discussed further below.

With respect to handle 50, and referring still to FIG. 1, the handle 50 includes a light 52 that illuminates the interior of the basket 20 by directing light into the open top 26 of the basket 20. The light 52 therefore allows a user to readily see any contents within the basket 20, but, as discussed below, the light 52 also provides additional visibility to the surroundings around the basket 20.

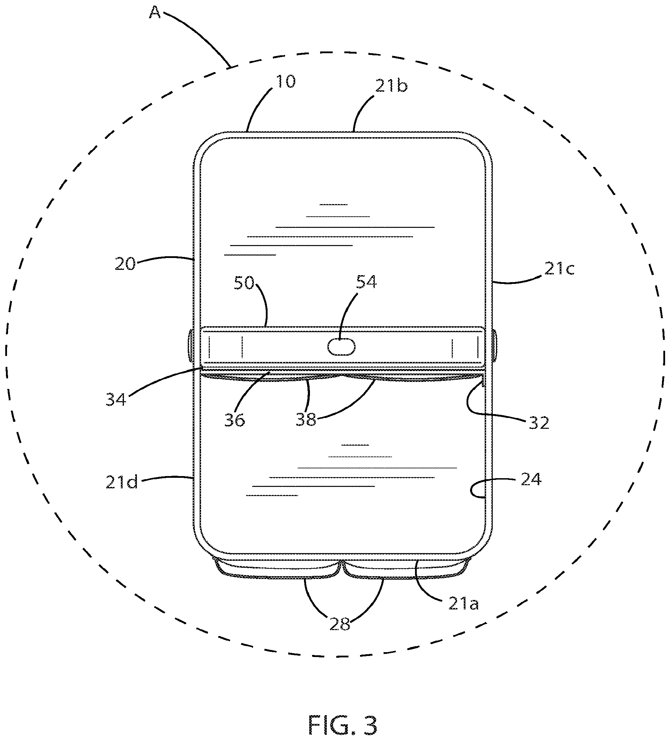

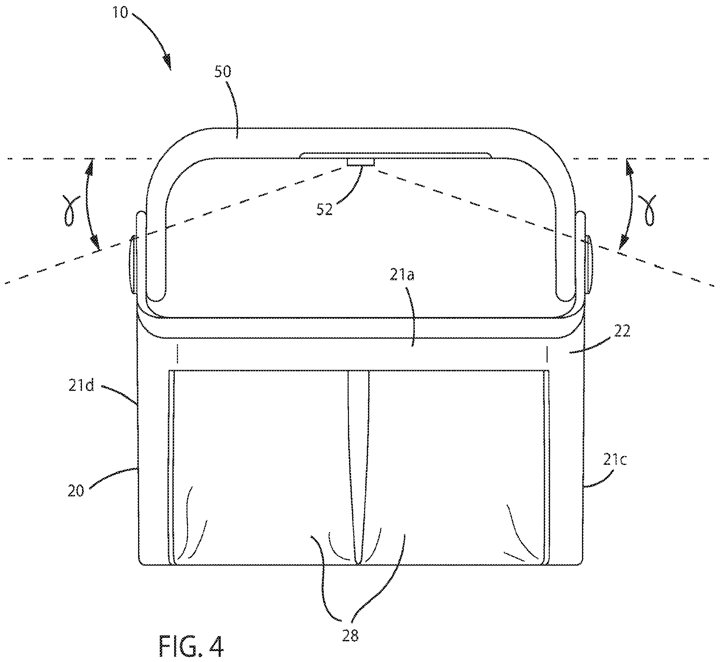

Turning now specifically to the light 52 of the present invention, in the exemplary embodiment shown in FIG. 1, the light 52 comprises a light source, such as an LED bulb, which is surrounded by a diffuser which projects below the bottom surface of the handle 50. Accordingly, the light 52 not only directs light into the basket 20, but with the diffuser, the light 52 also directs light around the basket 20. In particular, and referring now to FIGS. 3 and 4, because of the position of the diffuser, light is directed away from the light 52 at an angle, δ, relative to a horizontal direction such that an area A around the basket 20 is directly illuminated by the light 52. According to some embodiments of the present invention, the area A illuminated by the light 52 extends a distance of between about 10 inches to about 60 inches horizontally away from the light 52, between about 20 to about 50 inches horizontally away from the light 52, between about 30 to about 40 inches horizontally away from the light 52, between about 35 to about 40 inches horizontally away from the light 52, and preferably about 39 inches horizontally away from the light 52. Of course, the particular angle, δ, and distance of the area A of illumination provided by the light 52 is readily adjustable by a person of ordinary skill in the art, but in any event, in the present invention, the light 52 shines outward and downward, without shining upward, to illuminate the surroundings to assist, for example, in changing a diaper.

Furthermore, according to some embodiments of the present invention, the light 52 produces less than or equal to about 50 lumens, less than or equal to about 40 lumens, less than or equal to about 30 lumens, less than or equal to about 20 lumens, and, in one particular embodiment, the light 52 produces about 16 lumens. Because of the relatively low level of lumens produced by the light 52, the light 52 is less likely to disturb a tired or sleeping infant when the light 52 is on.

The light 52 is turned on/off by use of a switch 54 positioned on the top of the handle 50. In the exemplary embodiment shown in FIG. 1, the switch 54 is a touch switch that operates simply by being touched by a user, as compared to a mechanical switch which requires a user to physically move a portion of the switch. In this way, there is no clicking noise created when the switch 54 is activated and the light 52 is turned on/off, thus reducing the potential of disturbing a tired or sleeping infant. It is further contemplated that the light 52 also automatically turns off after a predetermined time. For example, in some embodiments, the light 52 turns off after about 10 minutes. Although not expressly shown, it should be understood that the handle 50 further includes an internal compartment that houses a power source to provide power to the light 52 as well as a CPU to control when the light 52 turns on and off according to a user touching the switch 54, a predetermined timer, or some other similarly programed instructions.

Referring now to the basket 20 in particular, the sides 21 of the basket 20 collectively define an exterior surface 22 and an interior surface 24 of the basket 20. In this exemplary embodiment, the front side 21a includes two exterior pockets 28 positioned along the exterior surface 22 of the basket 20. Of course, it is contemplated that any number of exterior pockets having any number of different sizes and shapes can be positioned anywhere around the exterior surface 22 of the basket 20.

The exemplary diaper caddy 10 of the present invention is designed to hold at least 30 lbs, but is preferably made of lightweight, semi-rigid material such that the diaper caddy 10 makes minimal noise when set down or moved. To this end, the sides 21 of the basket 20 are preferably made of a thin polyethylene board surrounded by a fabric liner. The polyethylene board is able to flex and partially deform while still providing sufficient strength to substantially maintain the overall shape of the basket 20 when loaded, and the fabric liner provides sound damping as well as an aesthetically appealing finish. Unlike the basket 20, the handle 50 is comprised of a rigid materials, such as a hard plastic. The rigid structure of the handle 50, not only provides adequate housing for the power source and CPU described above, but also maintains the position of the light 52 relative to the basket 20 to ensure adequate illumination into the basket 20.

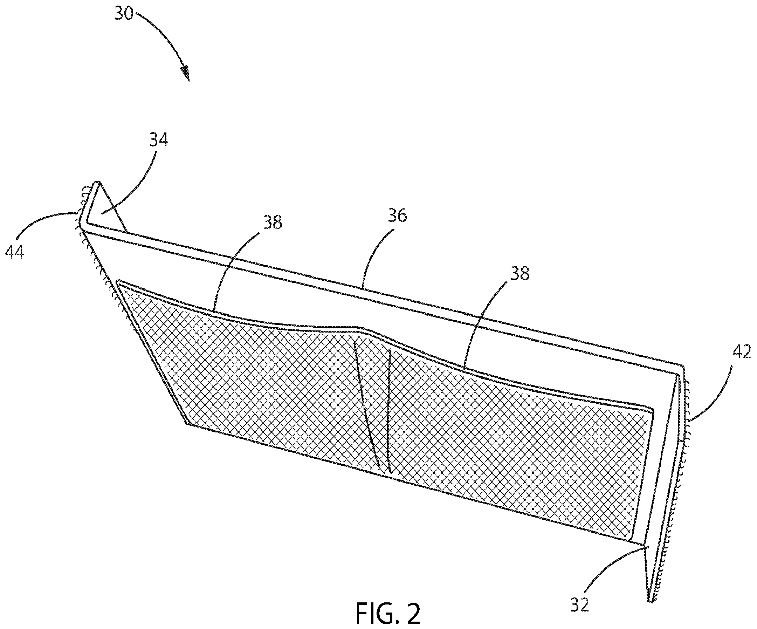

As previously mentioned, an insert 30 is positioned within the basket 20, and, in this exemplary embodiment, the insert 30 is operably connected to the right side 21c and the left side 21d along the interior surface 24 of the basket 20. In particular, a hook-and-loop fastener is used to removably secure the insert 30 to the interior surface 24. Referring now to FIG. 2, the insert 30 includes a first end 32, a second end 34 opposite the first end 32, and a central member 36 spanning between the first end 32 and the second end 34. Each of the ends 32, 34 includes one portion of a hook-and-loop fastener, which in this embodiment is the hook portion 42, 44.

Referring once again to FIG. 1, the right side 21c of the basket 20 includes a loop portion 46 of a hook-and-loop fastener along the interior surface 24 with the loop portion 46 configured to engage the hook portion 42 on the first end 32 of the insert 30. In particular, in the exemplary embodiment shown in FIG. 1, the loop portion 46 included on the interior surface 24 of the right side 21c of the basket 20 is a panel of tricot material, which the hook portion 42 on the first end 32 of the insert 30 readily grip to while still allowing the insert 30 to be removed repositioned and/or moved, thereby adjusting the respective sizes of the two resulting compartments on opposite sides of the insert 30. Although not shown, the hooks 44 on the second end 34 of the insert 30 similarly engage another panel of tricot material located on the interior surface 24 of the left side 21d of the basket 20.

The exemplary insert 30 shown in the Figures further includes two pockets 38 positioned on one side of the central member 36. Furthermore, and as perhaps best shown in FIG. 2, the two ends 32, 34 of the insert 30 are configured to bend in opposite directions relative to the central member 36 with the hook portions 42, 44 facing away from the central member 36. Of course, the particular configuration of the insert 30 described above is only an exemplary embodiment and other constructions are possible without departing from the spirit and scope of the present invention.

One of ordinary skill in the art will recognize that additional embodiments are also possible without departing from the teachings of the presently-disclosed subject matter. This detailed description, and particularly the specific details of the exemplary embodiments disclosed herein, is given primarily for clarity of understanding, and no unnecessary limitations are to be understood therefrom, for modifications will become apparent to those skilled in the art upon reading this disclosure and can be made without departing from the spirit and scope of the presently-disclosed subject matter.

## Claims

1. A diaper caddy, comprising:
a basket having an open top; and
a handle spanning the open top of the basket, the handle including a light source that directs light into the open top of the basket and around the basket.

2. The diaper caddy of claim 1, wherein the handle further includes a diffuser positioned adjacent to the light source, the diffuser configured to direct a portion of the light around the basket, and optionally, wherein the diffuser projects below a bottom surface of the handle.

3. The diaper caddy of either claim 1 or 2, wherein the light source produces about 16 lumens.

4. The diaper caddy of any preceding claim, wherein the handle further includes a touch switch that turns on the light source.

5. The diaper caddy of any preceding claim, wherein the light source automatically shuts off after a predetermined time, and optionally, wherein the predetermined time is about 10 minutes.

6. The diaper caddy of any preceding claim, wherein the basket is comprised of a semi-rigid material surrounded by a fabric liner, and optionally, wherein the handle is comprised of a rigid material.

7. The diaper caddy of any preceding claim, further comprising an insert positioned within the basket, the insert removably secured to the basket, and optionally, wherein the insert is removably secured to two sides of the basket by hook-and-loop fasteners.

8. A diaper caddy, comprising:
a basket having an open top; and
a handle spanning the open top of the basket, the handle including a light source that directs light into the open top of the basket and a diffuser positioned adjacent to the light source to direct a portion of the light around the basket.

9. The diaper caddy of claim 8, wherein the light source produces about 16 lumens.

10. The diaper caddy of either claim 8 or 9, wherein the handle further includes a touch switch that turns on the light source.

11. The diaper caddy of any of claims 8 to 10, wherein the basket is comprised of a semi-rigid material surrounded by a fabric liner, and optionally, , wherein the handle is comprised of a rigid material.

12. The diaper caddy of any of claims 8 to 11, further comprising an insert positioned within the basket, the insert removably secured to the basket.

13. A diaper caddy, comprising:
a basket having an open top; and
a handle spanning the open top of the basket, the handle including a light source that directs light into the open top of the basket, a diffuser positioned adjacent to the light source to direct a portion of the light around the basket, and a touch switch that turns on the light source; and
wherein the light source produces about 16 lumens.

14. The diaper caddy of claim 13, wherein the basket is comprised of a semi-rigid material surrounded by a fabric liner.

15. The diaper caddy of claim 14, further comprising an insert positioned within the basket, the insert removably secured to the basket.
